# FASCICULE DE BREVET EUROPEEN

(11) **EP 0 532 623 B1**
(45) Date de publication et mention de la délivrance du brevet: **07.09.1994**
(21) Numéro de dépôt: 91911120.3
(22) Date de dépôt: 06.06.1991
(51) Int. Cl.: A61F 2/36

(54) **PROTHESE DE HANCHE**
HÜFTPROTHESE
HIP PROSTHESIS

(30) Priorité: 07.06.1990 FR 9007374
(43) Date de publication de la demande: 24.03.1993
(73) Titulaire: ALLO PRO AG, 6340 Baar (CH)
(72) Inventeur: GODEAU, Denis, F-35800 Dinard (FR); ROLLAND, Jean-Jacques, F-22100 Dinan (FR); MENOU, Patrick, F-35510 Cesson-Sévigné (FR); CANCIANI, Jean-Pierre, F-35132 Vézin-le-Coquet (FR); LEBRET, Hervé, (FR); FERON, Franck, F-35760 Saint-Grégoire (FR); DELARUE, Patrice, F-22100 Dinan (FR); TRICLOT, Philippe, F-35300 Fougères (FR)
(74) Mandataire: Fournier, Michel Robert Marie
(86) Numéro de dépôt international: FR9100450
(87) Numéro de publication internationale: WO9118560

(56) Documents cités:
- EP-A- 234 358
- DE-A- 3 247 726
- DE-U- 8 213 101
- FR-A- 2 618 667
- FR-A- 2 619 707
- US-A- 4 546 501

## Description

La présente invention concerne une prothèse fémorale, et plus particulièrement une prothèse comprenant une queue d'ancrage dans la diaphyse fémorale et un col, lequel comporte une terminaison classique, par exemple un cône morse, pour sa liaison avec une sphère de remplacement de la tête fémorale.

La mise en place d'implant fémoral est devenue une intervention très courante, qui a de ce fait suscité d'importants travaux de recherche orientés sur la conception de modèles standardisés. Bien entendu, cette standardisation a obligé à s'écarter assez nettement des formes anatomiques, en accentuant les problèmes de fixation de la queue d'ancrage dans la partie restante du fémur, si bien qu'à ce jour, aucune solution réellement satisfaisante n'est disponible. En ce qui concerne les prothèses cimentées, on ne peut éviter une dégradation du ciment et/ou de l'os avec le temps, alors que les prothèses sans ciment posent un problème de fixation primaire entraînant des douleurs.

Par ailleurs, ce manque de congruence découlant de la standardisation se traduit également par des difficultés pour le chirurgien dans le positionnement précis de la prothèse, notamment pour reproduire l'antéversion que présente naturellement le col du fémur, et pour empêcher la queue d'ancrage de se mettre en varus, avec appui de l'extrémité distale sur la corticale interne du fémur.

Le but de la présente invention est de concevoir une nouvelle prothèse apportant une meilleure réponse globalc à l'ensemble de ces problèmes.

Cette nouvelle prothèse comporte classiquement une queue d'ancrage dans la diaphyse fémorale, un col raccordable à une sphère de remplacement de la tête fémorale, une collerette d'appui sur le bord sectionné du fémur, et elle est "anatomique", en ce sens qu'un implant droit est la reproduction en miroir d'un implant gauche sans qu'ils soient interchangeables.

Par le document DE-A-32 47 726, on connaît une prothèse fémorale de ce type, dans laquelle la tige est de section sensiblement circulaire dans sa partie inférieure, et de section sensiblement elliptique dans sa partie supérieure. Elle présente en vue latérale une courbure en forme de S correspondant à celle du canal médullaire.

D'autre part, dans cette partie supérieure, l'axe longitudinal de la section elliptique fait un angle d'environ 15° avec le plan latéral passant par l'axe théorique de la tige. Pour obtenir un bon blocage de la prothèse, la face ventrale de la partie supérieure est plane, tandis que la face dorsale est convexe. D'autres moyens antirotatoires sont prévus, notamment un aileron supéroexterne et une configuration en quadrilatère de l'extrémité inférieure de la tige.

Dans une prothèse fémorale conforme à l'invention comprenant une queue d'ancrage dans la diaphyse fémorale et un col raccordable à une sphère de remplacement de la tête fémorale :
une collerette d'appui est prévue entre la queue d'ancrage et le col ;
la queue d'ancrage est incurvée vers l'intérieur dans sa partie supérieure de manière que son axe théorique au niveau de la collerette fasse un angle d'environ 135° avec le même axe dans la partie intermédiaire ; et présente une courbure régulière à concavité postérieure sur au moins sa partie au-dessus de son tiers inférieur ;
la partie supérieure de la queue d'ancrage a une section croissant de bas en haut et de forme ovoïde avec la pointe dirigée vers l'intérieur et l'autre extrémité dirigée vers l'extérieur ; et présente une torsion, consistant en un vrillage progressif de bas en haut dans le sens de l'antéversion du col, de telle manière que le grand axe de la section en son sommet soit décalé angulairement par rapport au grand axe de la section à sa base, autour de l'axe théorique, de la valeur de l'antéversion, le col étant orienté exactement dans le prolongement de la queue d'ancrage.

Il est prévu une version à cimenter de la prothèse, et une version sans ciment, qui se distingue de la première nommée par un léger surdimentionnement de la queue d'ancrage à tailles égales (pour un blocage par impaction), et par un aménagement de sa paroi pour lui procurer un état de surface approprié.

L'invention sera mieux comprise à l'aide des explications qui vont suivre, et des dessins joints, parmi lesquels :
la Fig. 1 est une vue de face d'une prothèse selon l'invention, et
la Fig. 2 est une vue de profil de la prothèse de la Fig. 1.

On a représenté sur les dessins une prothèse droite qui comporte classiquement une queue d'ancrage 1, un col 2 et une collerette 3. Le positionnement et l'inclinaison de celle-ci montrent qu'il s'agit d'une prothèse destinée à être placée dans un fémur sectionné au-dessus du grand trochanter.

L'étude de la prothèse a été menée en vue de plusieurs objectifs principaux, à savoir : obtenir un remplissage optimal de la métaphyse fémorale avec la queue d'ancrage, tout en préservant sa facilité d'insertion; assurer une bonne répartition des contraintes transmises de l'implant vers l'os, en les favorisant dans les régions naturellement plus sollicitées, c'est-à-dire au niveau du calcar; établir un blocage primaire limitant au maximum les risques d'enfoncement et de rotation axiale; obtenir une rigidité et une robustesse satisfaisantes.

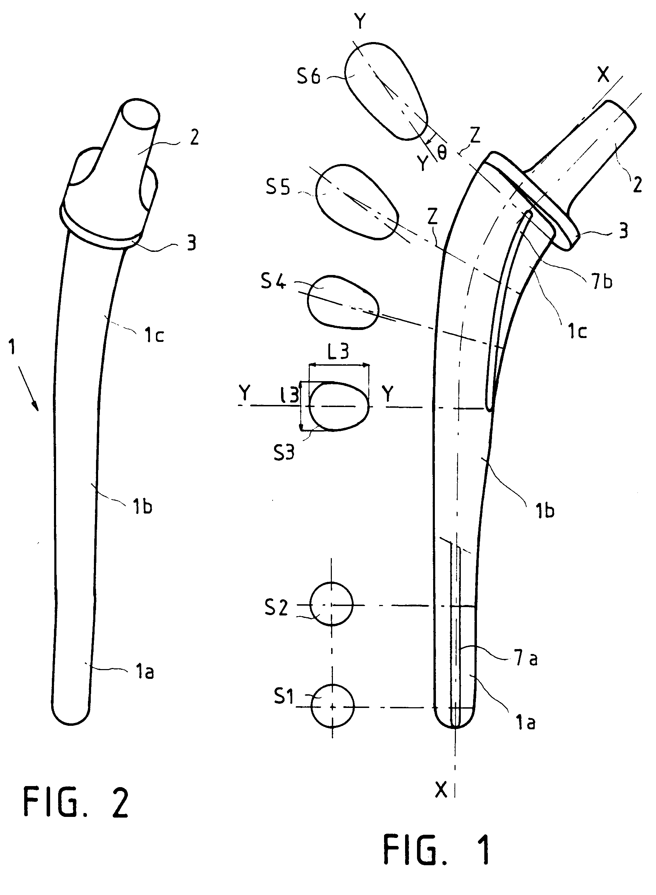

Dans sa forme générale, la queue d'ancrage 1 présente une courbure régulière sur la plus grande partie de sa longueur, qui reproduit sensiblement la concavité d'avant en arrière du fémur. Cette courbure apparaît à la Fig. 2, où elle est interrompue environ au niveau du tiers inférieur, du fait que dans l'exemple représenté, une contre-courbure est ménagée à cet endroit, dans le but de désaxer légèrement vers l'avant la partie inférieure, soit d'environ 2° en pratique. Cette contre-courbure permet d'éviter les contacts, toujours douloureux, de l'extrémité distale de la queue d'ancrage 1 contre la corticale.

En vue de face, Fig. 1, la queue d'ancrage 1 est extérieurement sensiblement rectiligne, ne s'incurvant que près de son sommet, alors qu'intérieurement, elle présente une concavité à partir du tiers inférieur, qui s'accentue vers le haut, en même temps que la largeur croît.

Pour plus de détail, on peut considérer que la queue d'ancrage 1 est constituée sur sa hauteur de trois parties distinctes de longueur sensiblement égales. La partie inférieure 1a est sensiblement rectiligne et sa section est circulaire et pratiquement uniforme, figurée par les deux cercles repérés S1 et S2. Dans la partie médiane 1b, laquelle est également sensiblement rectiligne, la section évolue progressivement de la forme circulaire S2 vers une forme ovoïde S3 à la base de la partie supérieure 1c. La section S3 est allongée transversalement et sa pointe est dirigée vers l'intérieur. La largeur 13 de S3 n'est que légèrement supérieure au diamètre de S2, alors que sa longueur L3 s'est accrue dans un rapport d'environ 1,5.

Dans la partie supérieure 1c, la section évolue progressivement de S3 vers S6 au niveau de la jonction avec la collerette 2, en s'accroissant dans un rapport d'environ 1,7 pour sa longueur, et d'environ 1,3 pour sa largeur. D'autre part, la partie supérieure 1c s'incurve progressivement vers l'intérieur, de sorte qu'au niveau de la collerette 2, le prolongement de son axe théorique X-X, Fig. 1, fasse un angle d'environ 135° avec celui de la partie intermédiaire 1b.

Selon une caractéristique importante de l'invention, la partie supérieure 1c a, de plus, une configuration légèrement vrillée (présentant par exemple une héli-torsion), comme l'illustrent les sections à différents niveaux S3 à S6, dont les axes longitudinaux Y-Y sont progressivement décalés autour de l'axe théorique X-X par rapport au plan transversal illustré par les lignes Z. Au niveau de S6, l'angle de décalage ϑ est d'environ 12°, alors qu'il est nul au niveau de S3. Il est orienté dans le sens de l'antéversion du col (ou du prolongement de l'axe X-X).

Cette forme particulière de la queue d'ancrage 1 permet une orientation du col 2 exactement dans son prolongement. On améliore ainsi sa rigidité et sa robustesse. Dans l'exemple de réalisation montré, le col 2 est déporté vers l'intérieur par rapport à l'axe X-X, mais, en variante, on peut lui donner une position plus centrale par rapport à la section S6.

La collerette 3 est prévue pour prendre appui sur le bord sectionné du fémur. De préférence, comme montré dans les dessins, elle ne fait pas le tour complet de la queue d'ancrage 1, et elle est prédominante intérieurement. Sur l'avant et sur l'arrière, elle décroît en largeur vers l'extérieur, jusqu'à se fondre dans la paroi de la queue d'ancrage 1, de sorte qu'elle est inexistante sur le côté externe pour ne pas buter sur le grand trochanter. La collerette 3 exerce donc son action essentiellement au niveau du calcar, c'est-à-dire dans la région du fémur la plus sollicitée naturellement. A la Fig. 2, on remarquera une certaine inclinaison transversale de la collerette 3. Dans cette forme de réalisation, cette inclinaison est déterminée de telle manière que la surface inférieure de la collerette 3 porte intégralement sur la tranche de section du col (voie postérieure).

Lorsqu'une prothèse selon l'invention est insérée dans le canal médullaire du fémur, elle se place d'elle-même pratiquement dans la position adéquate, guidée par le jeu conjugué de ses courbures et de la forme évolutive de sa section avec la paroi interne du canal médullaire. Par rapport à l'axe bi-condylien, en raison de la configuration vrillée de la queue d'ancrage 1, l'antéversion naturelle du col est reproduite. A noter que celle-ci n'est pas apparente dans les dessins, du fait que les Figs. 1 et 2 ne sont pas vues perpendiculairement aux plans sagital et frontal anatomiques, respectivement, mais que c'est l'axe Y-Y de la section S3 qui a été choisi comme référence transversale.

Par la forme évolutive de la section de la queue d'ancrage 1, on privilégie le remplissage dans la partie proximale du canal médullaire, afin que les contraintes de l'implant vers l'os prédominent dans la région naturellement la plus sollicitée du fémur. On assure un positionnement correct dans le plan frontal, et on réalise un véritable "auto-blocage" sans qu'il soit nécessaire d'encastrer l'implant en force.

A la Fig. 1, les références 7a, 7b indiquent des rainures longitudinales ménagées dans la paroi de la queue d'ancrage 1, dont l'une 7a n'est représentée que partiellement à des fins de clarté du dessin. Ces rainures ont pour but de contribuer au blocage en rotation. Dans la version à cimenter, leur action est à deux niveaux : elles sont efficaces au niveau du blocage primaire en se remplissant de ciment dans les zones ou celui-ci est présent, c'est-à-dire principalement au-delà de la région proximale. Dans celle-ci, là où il y a contact intime entre la paroi de la queue d'ancrage et l'os, elles forment des vides propices à l'ostéogénèse.

Dans la version sans ciment, la paroi de la queue d'ancrage 1 présente par ailleurs un état de surface approprié, par exemple un quadrillage en relief obtenu par un revêtement de réseaux grillagés en plusieurs épaisseurs.

Pour couvrir l'ensemble des besoins, plusieurs tailles de prothèse sont nécessaires. En pratique, leur nombre peut être limité à 9. Quant au matériau de fabrication, ce peut être du titane, un alliage de titane, de l'acier inoxydable, ou tout autre biomatériau compatible.

## Revendications

1. Prothèse fémorale comprenant une queue (1) d'ancrage dans la diaphyse fémorale et un col (2) raccordable à une sphère de remplacement de la tête fémorale, dans laquelle :
une collerette d'appui (3) est prévue entre la queue d'ancrage (1) et le col (2) ;
la queue d'ancrage (1) est incurvée vers l'intérieur dans sa partie supérieure (1c) de manière que son axe théorique (X-X) au niveau de la collerette (3) fasse un angle d'environ 135° avec le même axe dans la partie intermédiaire (1b) ; et présente une courbure régulière à concavité postérieure sur au moins sa partie au-dessus de son tiers inférieur (1a) ;
la partie supérieure (1c) de la queue d'ancrage (1) a une section croissant de bas en haut et de forme ovoïde avec la pointe dirigée vers l'intérieur et l'autre extrémité dirigée vers l'extérieur ; et présente une torsion, consistant en un vrillage progressif de bas en haut dans le sens de l'antéversion du col, de telle manière que le grand axe (Y-Y) de la section (S6) en son sommet soit décalé angulairement par rapport au grand axe de la section (S3) à sa base, autour de l'axe théorique (X-X), de la valeur de l'antéversion, le col (2) étant orienté exactement dans le prolongement de la queue d'ancrage (1).

2. Prothèse selon la revendication 1, caractérisée en ce que la section ovoïde de la partie supérieure (1c) de la queue d'ancrage (1) évolue de bas en haut vers une forme plus allongée.

3. Prothèse selon la revendication 2, caractérisée en ce que dans la partie supérieure (1c) de la queue d'ancrage (1), la section évolue progressivement de (S3) vers (S6) au niveau de la jonction avec la collerette (2), en s'accroissant dans un rapport d'environ 1,7 pour sa longueur et d'environ 1,3 pour sa largeur.

4. Prothèse selon l'une des revendications 1 à 3, caractérisée en ce que la partie supérieure (1c) de la queue d'ancrage (1) présente une héli-torsion.

5. Prothèse selon la revendication 1 ou 2, caractérisée en ce que la section de la queue d'ancrage (1) est également ovoïde dans sa partie médiane (1b).

6. Prothèse selon l'une des revendications 1 à 5, caractérisée en ce que le décalage angulaire ϑ entre le grand axe (Y-Y) de la section (S6) et celui de la section (S3) est d'environ 12°.

7. Prothèse selon l'une des revendications 1 à 6, caractérisée en ce que la section de la queue d'ancrage (1) dans sa partie inférieure (1a) est sensiblement circulaire et uniforme.

8. Prothèse selon l'une des revendications 1 à 7, caractérisée en ce que la partie inférieure (1a) de la queue d'ancrage (1) est légèrement désaxée vers l'avant par rapport à la partie médiane (1b).

9. Prothèse selon l'une ds revendications 1 à 8, caractérisée en ce que le col (2) est déporté vers l'intérieur par rapport à l'axe théorique (X-X) de la queue d'ancrage (1).

10. Prothèse selon l'une des revendications 1 à 9, caractérisée en ce que la collerette (3) est prédominante intérieurement et inexistante extérieurement.

11. Prothèse selon l'une des revendications 1 à 10, caractérisée en ce que des rainures longitudinales (7a, 7b) sont ménagées dans la paroi de la queue d'ancrage (1).

12. Prothèse selon l'une des revendications 1 à 11, caractérisée en ce que la paroi de la queue d'ancrage (1) présente un quadrillage en relief.

## Claims

1. A femoral prosthesis comprising a shaft (1) for anchoring in the femoral diaphysis and a neck (2) that can be connected to a sphere replacing the head of the femur, in which:
a bearing flange (3) is provided between the anchoring shaft (1) and the neck (2);
the anchoring shaft (1) is curved inwards in its upper portion (1c) such that its theoretical axis (X-X) in the area of the flange (3) makes an angle of about 135° with the same axis in the intermediate portion (1b); and has a regular curvature with rear concavity over at least its portion above its lower third (1a);
the upper portion (1c) of the anchoring shaft (1) has a section increasing towards the top and is ovoid in form with the tip pointing inwards and the other end pointing outwards; and has torsion, consisting of gradual twisting towards the top in the direction of anteversion of the neck, such that the major axis (Y-Y) of the section (S6) at its highest point is offset angularly relative to the major axis of the section (S3) at its base, around the theoretical axis (X-X), by the value of the anteversion, the neck (2) being oriented exactly in the extension of the anchoring shaft (1).

2. A prosthesis according to Claim 1, characterised in that the ovoid section of the upper part (1c) of the anchoring shaft (1) develops upwards into a more elongated shape.

3. A prosthesis according to Claim 2, characterised in that in the upper portion (1c) of the anchoring shaft (1) the section develops gradually from (S3) to (S6) in the area of the junction with the flange (2), getting larger in a ratio of around 1.7 for its length and around 1.3 for its width.

4. A prosthesis according to one of Claims 1 to 3, characterised in that the upper portion (1c) of the anchoring shaft (1) has a helical torsion.

5. A prosthesis according to Claim 1 or 2, characterised in that the section of the anchoring shaft (1) is also ovoid in its median portion (1b).

6. A prosthesis according to one of Claims 1 to 5, characterised in that the angular offset ϑ between the major axis (Y-Y) of the section (S6) and that of the section (S3) is around 12°.

7. A prosthesis according to one of Claims 1 to 6, characterised in that the section of the anchoring shaft (1) in its lower portion (1a) is substantially circular and uniform.

8. A prosthesis according to one of Claims 1 to 7, characterised in that the lower portion (1a) of the anchoring shaft (1) is slightly out-of-line towards the front relative to the median portion (1b).

9. A prosthesis according to one of Claims 1 to 8, characterised in that the neck (2) is shifted inwards relative to the theoretical axis (X-X) of the anchoring shaft (1).

10. A prosthesis according to one of Claims 1 to 9, characterised in that the flange (3) is predominant on the inside and non-existent on the outside.

11. A prosthesis according to one of Claims 1 to 10, characterised in that longitudinal grooves (7a, 7b) are contrived in the wall of the anchoring shaft (1).

12. A prosthesis according to one of Claims 1 to 11, characterised in that the wall of the anchoring shaft (1) has a grid pattern in relief.

## Patentansprüche

1. Oberschenkelprothese mit einem Schaft (1) zur Verankerung in der Femurdiaphyse sowie einem Hals (2), der mit einer Ersatzkugel für den Femurkopf verbunden werden kann, bei der:
- ein Anlagebund (3) zwischen dem Verankerungsschaft (1) und dem Hals (2) vorgesehen ist;
- der Verankerungsschaft (1) in seinem oberen Teil (1c) so nach innen gebogen ist, daß seine theoretische Achse (X-X) auf Höhe des Bundes (3) mit der gleichen Achse in dem Zwischenteil (1b) einen Winkel von etwa 135° bildet, und wenigstens an seinem Teil über dem unteren Drittel (1a) eine regelmäßige Krümmung mit hinterer Konkavität aufweist;
- der obere Teil (1c) des Verankerungsschafts (1) einen von unten nach oben zunehmenden, ovalen Querschnitt aufweist, wobei die Spitze nach innen und das andere Ende nach außen gerichtet ist, und eine Verwindung aufweist, die in einer in Richtung der Anteversion des Halses von unten nach oben zunehmenden Verdrehung besteht, so daß die Hauptachse (Y-Y) des Querschnitts (S6) an ihrem Scheitel bezüglich der Hauptachse des Querschnitts (S3) an seiner Basis um die theoretische Achse (X-X) mit dem Wert der Anteversion winkelversetzt ist, wobei der Hals (2) exakt in der Verlängerung des Verankerungsstiels (1) orientiert ist.

2. Prothese nach Anspruch 1, dadurch gekennzeichnet, daß sich der ovale Querschnitt des oberen Teils (1c) des Verankerungsschafts (1) von unten nach oben zu einer länglicheren Form entwickelt.

3. Prothese nach Anspruch 2, dadurch gekennzeichnet, daß sich der Querschnitt in dem oberen Teil (1c) des Verankerungsschafts (1) fortschreitend von (S3) nach (S6) auf Höhe der Verbindung mit dem Bund (2) ändert, wobei er in einem Verhältnis von etwa 1,7 für die Länge und etwa 1,3 für seine Breite zunimmt.

4. Prothese nach einem der Ansprüche 1 bis 3, dadurch gekennzeichnet, daß der obere Teil (1c) des Verankerungsschafts (1) eine schraubenförmige Verwindung aufweist.

5. Prothese nach Anspruch 1 oder 2, dadurch gekennzeichnet, daß der Querschnitt des Verankerungsschafts (1) auch in seinem Mittelteil (1b) oval ist.

6. Prothese nach einem der Ansprüche 1 bis 5, dadurch gekennzeichnet, daß der Winkelversatz ϑ zwischen der Hauptachse (Y-Y) des Querschnitts (S6) und dem des Querschnitts (S3) etwa 12° beträgt.

7. Prothese nach einem der Ansprüche 1 bis 6, dadurch gekennzeichnet, daß der Querschnitt des Verankerungsschafts (1) in seinem unteren Teil (1a) im wesentlichen kreisförmig und gleichmäßig ist.

8. Prothese nach einem der Ansprüche 1 bis 7, dadurch gekennzeichnet, daß der untere Teil (1a) des Verankerungsschafts (1) bezüglich des Mittelteils (1b) leicht nach vorne achsenversetzt ist.

9. Prothese nach einem der Ansprüche 1 bis 8, dadurch gekennzeichnet, daß der Hals (2) bezüglich der theoretischen Achse (X-X) des Verankerungsschafts (1) nach innen verlagert ist.

10. Prothese nach einem der Ansprüche 1 bis 9, dadurch gekennzeichnet, daß der Bund (3) innen vorherrschend und außen nicht existent ist.

11. Prothese nach einem der Ansprüche 1 bis 10, dadurch gekennzeichnet, daß Längsnuten (7a, 7b) in der Wand des Verankerungsschafts (1) vorgesehen sind.

12. Prothese nach einem der Ansprüche 1 bis 11, dadurch gekennzeichnet, daß die Wand des Verankerungsschafts (1) ein erhabenes Raster aufweist.
